# EUROPÄISCHE PATENTSCHRIFT

(11) **EP 0 002 678 B1**
(45) Veröffentlichungstag und Bekanntmachung des Hinweises auf die Patenterteilung: **30.12.1981**
(21) Anmeldenummer: 78101534.2
(22) Anmeldetag: 04.12.1978
(51) Int. Cl.: C07D 401/12, A01N 43/48, A01N 43/64

(54) **Azolylalkyl-pyridinyl-äther, Verfahren zu ihrer Herstellung sowie ihre Verwendung als Fungizide**
Azolylalkyl-pyridinyl ethers, process for their preparation and their use as fungicides
Ethers azolylalkyl-pyridinyl, leur procédé de préparation ainsi que leur utilisation comme fongicides

(30) Priorität: 16.12.1977 DE 2756269
(43) Veröffentlichungstag der Anmeldung: 11.07.1979
(73) Patentinhaber: BAYER AG, 51368 Leverkusen (DE)
(72) Erfinder: Stetter, Jörg, Dr., D-5600 Wuppertal 1 (DE); Kraatz, Udo, Dr., D-5090 Leverkusen 1 (DE); Büchel, Karl Heinz, Prof. Dr., D-5600 Wuppertal 1 (DE); Frohberger, Paul-Ernst, Dr., D-5090 Leverkusen 1 (DE); Brandes, Wilhelm, Dr., D-5653 Leichlingen 1 (DE); Paul, Volker, Dr., D-5650 Solingen 1 (DE)

## Beschreibung

Die vorliegende Erfindung betrifft neue Azolylalkylpyridinyläther, mehrere Verfahren zu ihrer Herstellung sowie ihre Verwendung als Fungizide.

Es ist bereits bekannt geworden, dass Tritylimidazole und -1,2,4-triazole, wie 1-Triphenylmethylimidazol und 1-Tripheny)methy!-1,2,4-triazol,einegutefungizideWirksamkeitbesitzen (vgl. US-PS Nr. 3321366 und DE-OS Nr. 1795249). Weiterhin sind in der Literatur 1,2,4-Triazolderivate von Phenoxyketonen beschrieben (vgl. z.B. DE-OS Nr. 2406665), die teilweise über eine sehr gute fungizide Wirksamkeit verfügen. Die Wirksamkeit der vorbekannten Verbindungen ist jedoch insbesondere bei niedrigen Aufwandmengen und -konzentrationen, nicht immer ganz befriedigend.

Es wurden nun als neue Verbindungen die Azolylalkylpyridinyläther der Formel 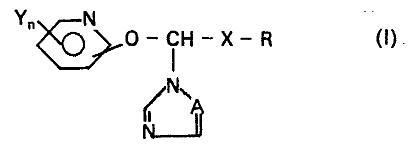 in welcher
Afür die CH -Gruppe oder ein Stickstoffatom steht,
R für Alkyl mit 1 bis 4 Kohlenstoffatomen steht,
X für eine Ketogruppe oder eine CH(OH)-Gruppierung steht,
Y für Halogen, Alkyl mit 1 bis 4 Kohlenstoffatomen oder für Cyano steht, und
n für die Zahlen 0, 1, 2 oder 3 steht,

und deren Säureadditionssalze sowie Metallsalzkomplexe gefunden. Sie weisen starke fungizide Eigenschaften auf.

Diejenigen Verbindungen der Formel (I), in welchen X für die CH(OH)-Gruppe steht, besitzen zwei asymmetrische Kohlenstoffatome; sie können deshalb in den beiden geometrischen Isomeren (erythro- und threoform) vorliegen, die in unterschiedlichen Mengenverhältnissen anfallen können. In beiden Fällen liegen sie als optische Isomeren vor. Sämtliche Isomeren werden erfindungsgemäss beansprucht.

Weiterhin wurde gefunden, dass man die Azolylalkylpyridinyläther der Formel (I) erhält, wenn man
a) Azolylhalogenketone der Formel: 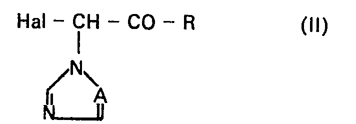
   in welcher
   A und R die oben angegebene Bedeutung haben, und
   Hal für Chlor oder Brom steht,
   mit Pyridinolen der Formel: 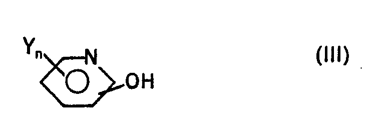
   in welcher
   Y und n die oben angegebene Bedeutung haben, in Gegenwart eines Säurebindemittels und gegebenenfalls in Gegenwart eines Verdünnungsmittels umsetzt, oder
b) Halogenätherketone der Formel: 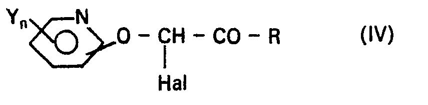 in welcher
   Hai, R, Y und n die oben angegebene Bedeutung haben,

   mit Imidazol oder 1,2,4-Triazol in Gegenwart eines Säurebindemittels und gegebenenfalls in Gegenwart eines Verdünnungsmittels umsetzt, oder
c) Dihalogenketone der Formel: 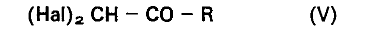 in welcher
   R und Hal die oben angegebene Bedeutung haben,

   mit Imidazol oder 1,2,4-Triazol und mit einem Pyridinol der Formel (III) in Gegenwart eines Säurebindemittels und gegebenenfalls in Gegenwart eines Verdünnungsmittels umsetzt und gegebenenfalls die nach den Verfahrensvarianten (a), (b) und
(c) erhaltenen Ketoderivate nach bekannten Methoden in üblicher Weise reduziert.

An die so erhaltenen Verbindungen der Formel (I) kann gegebenenfalls anschliessend eine Säure oder ein Metallsalz addiert werden.

Überraschenderweise zeigen die erfindungsgemässen Wirkstoffe eine erheblich höhere fungizide Wirksamkeit, insbesondere gegen Getreidekrankheiten, als die aus dem Stand der Technik bekannten Verbindungen 1 -Triphenytmethyjimidazol und 1-Triphenylmethyl-1,2,4-triazol, welches bekannte Stoffe gleicher Wirkungsrichtung sind. Die erfindungsgemässen Wirkstoffe stellen somit eine Bereicherung der Technik dar.

Verwendet man
1-Brom-3,3-dimethyl-1-(1,2,4-triazol-1-yl)-butan-2-on und
6-Chlor-pyridin-2-ol

als Ausgangsstoffe, so kann der Reaktionsablauf durch das folgende Formelschema wiedergegeben werden (Verfahrensvariante a):

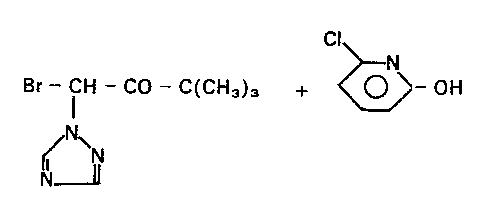

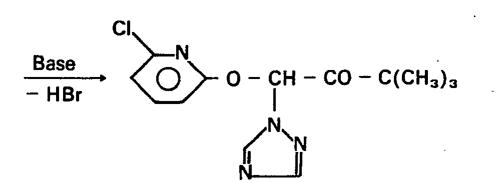

Verwendet man
1-Brom-1-(6-chlorpyridin-2-yloxy)-3,3-dimethylbutan-2-on

und Imidazol als Ausgangsstoffe, so kann der Reaktionsablauf durch das folgende Formelschema wiedergegeben werden (Verfahrensvariante b):

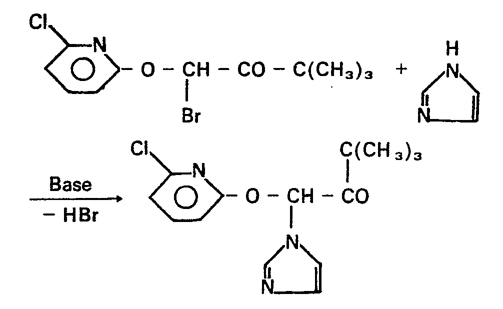

Verwendet man
6-Chlorpyridin-2-ol, Dichlorpinakolin und 1,2,4-Triazol

als Ausgangsstoffe, so kann der Reaktionsablauf durch das folgende Formelschema wiedergegeben werden (Verfahrensvariante c):

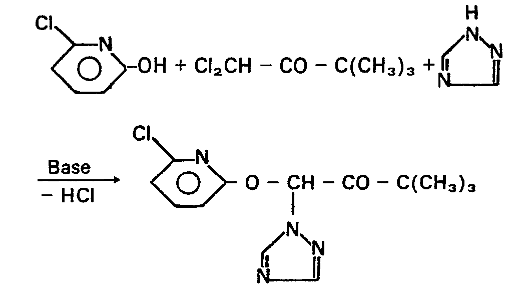

Verwendet man
1-(6-Ch)orpyridin-2-yloxy)-3,3-dimethyl-1-(1,2,4-triazol-1-yl)-butan-2-on

als Keton und Natriumborhydrid als Reduktionsmittel, so kann der Reaktionsablauf durch das folgende Formelschema wiedergegeben werden (Reduktion):

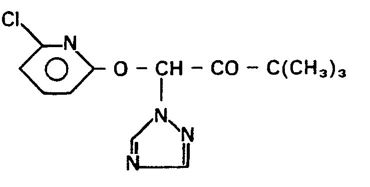

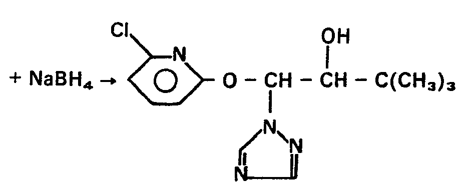

Die als Ausgangsstoffe für die Verfahrensvariante (a) zu verwendenden Azolylhalogenketone sind durch die Formel (II) definiert.

Die Azolylhalogenketone der Formel (II) sind noch nicht bekannt, können aber nach bekannten Verfahren hergestellt werden, indem man bekannte Halogenide (vgl. DT-OS Nr. 2613167 [Le A Nr. 17056]) der Formel: 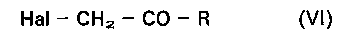 mit Imidazol oder 1,2,4-Triazol in Gegenwart eines Säurebindemittels, wie z.B. Kaliumcarbonat, und in Gegenwart eines inerten organischen Lösungsmittels, wie z.B. Aceton, bei Temperaturen zwischen 60 und 120°C umsetzt. Eines der beiden aktiven Wasserstoffatome wird anschliessend in üblicher Weise gegen Chlor oder Brom ausgetauscht.

Als Ausgangsstoffe der Formel (11) seien beispielsweise genannt:
1-Brom,3,3-dimethyl-1 -(1,2,4-triazol-1 -yl)-butan2-on
1-Chlor-3,3-dimethyl-1-(1,2,4-triazol-1-yl)-butan-2-on
1-Brom-3,3-dimethyl-1-imidazol-1-yl- butan-2-on
1-Chlor-3,3-dimethyl-1-imidazol-1-yl- butan-2-on
1-Brom-1-(1,2,4-triazol-1-yl)-propan-2-on
1-Brom-1-imidazol-1-yl-propan-2-on
1-Brom-3-methyl-1-(1,2,4-triazol-1-yl)-butan-2-on
1-Brom-1-imidazol-1-yl-3-methyl-butan-2-on.

Die ausserdem für die Verfahrensvariante (a) als Ausgangsstoffe zu verwendenden Pyridinole sind durch die Formel (III) definiert.

Die Pyridinole der Formel (III) sind allgemein bekannte Verbindungen der organischen Chemie.

Als Beispiele seien genannt:
2-Hydroxypyridin, 3-Hydroxypyridin, 4- Hydroxypyridin
2-Hydroxy-6-chlorpyridin, 2-Hydroxy-5-chlor- pyridin
2-Hydroxy-4-chlorpyridin, 2-Hydroxy-3-chlor- pyridin
2-Hydroxy-6-brompyridin, 2-Hydroxy-5-brom- pyridin
2-Hydroxy-4-brompyridin, 2-Hydroxy-3-brom- pyridin
2-Hydroxy-6-methylpyridin, 2-Hydroxy-5-methylpyridin
2-Hydroxy-4-methylpyridin, 2-Hydroxy-3-methylpyridin
2-Hydroxy-6-fluorpyridin, 2-Hydroxy-5-fluorpyridin
2-Hydroxy-4-fluorpyridin, 2-Hydroxy-3-fluorpyridin
3-Hydroxy-2-chlorpyridin, 3-Hydroxy-2-brompyridin
3-Hydroxy-2-fluorpyridin, 3-Hydroxy-2-jodpyridin
3-Hydroxy-6-chlorpyridin
3-Hydroxy-5-chlorpyridin, 4-Hydroxy-2-chlorpyridin,
4-Hydroxy-3-chlorpyridin.

Die für die Verfahrensvariante (b) aIsAusgangsstoffe zu verwendenden Halogenätherketone sind durch die Formel (IV) definiert.

Die Halogenätherketone der Formel (IV) sind noch nicht bekannt, können aber nach bekannten Verfahren hergestellt werden, indem man Pyridinole der Formel (III) mit Halogenketonen der Formel (VI) in Gegenwart eines Säurebindemittels, wie z.B. Kaliumcarbonat, und in Gegenwart eines inerten organischen Lösungsmittels, wie z.B. Aceton, bei Temperaturen zwischen 60 und 120°C umsetzt. Eines der beiden aktiven Wasserstoffatome wird anschliessend in üblicher Weise gegen Chlor oder Brom ausgetauscht.

Als Ausgangsstoffe der Formel (IV) seien beispielsweise genannt:
1 Brom-3,3-dimethyl-1 -pyridin-2-ylbutan-2-on
1-Brom-3,3-dimethyl-1 -pyridin-3-ylbutan-2-on
1-Brom-3,3-dimethyl-1-pyridin-4-ylbutan-2-on
1-Brom-1-(6-chlorpyridin-2-yl)-3,3-dimethylbutan-2-on
1-Chlor-1-(6-chlorpyridin-2-yl)-3,3-dimethylbutan-2-on
1-Brom-1-(5-chlorpyridin-2-yl)-3,3-dimethylbutan-2-on
1 -Brom-1 (2-chlorpyridin-3-yl)-3,3-dimethylbutan-2-on
1-Brom-1-(2-brompyridin-3-yl)-3,3-dimethylbutan-2-on
1 -Brom-1(3,4,5-trichlorpyridin-2-yl)-3,3-dimethylbutan-2-on.

Die für die Verfahrensvariante (c) als Ausgangsstoffe zu verwendenden Dihalogenketone sind durch die Formel (V) definiert.

Die Dihalogenketone der Formel (V) sind allgemein bekannte Verbindungen der organischen Chemie. Als Beispiele seien genannt: Dichlorpinakolin, Dibrompinakolin.

Für die erfindungsgemässen Umsetzungen gemäss Verfahrensvarianten (a) und (b) kommen als Verdünnungsmittel inerte organische Lösungsmittel infrage. Hierzu gehören vorzugsweise Ketone, wie Diäthylketon, insbesondere Aceton und Methyläthylketon; Nitrile, wie Propionitril, insbesondere Acetonitril; Alkohole, wie Äthanol oder Isopropanol; Äther, wie Tetrahydrofuran oder Dioxan; Benzol; Formamide, wie insbesondere Dimethylformamid; und halogenierte Kohlenwasserstoffe.

Die Umsetzungen nach Verfahren (a) und (b) werden in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triäthylamin, Dimethylbenzylamin; oder wie Pyridin und Diazabicyclooctan.

Bei der Verfahrensvariante (b) kann auch ein entsprechender Überschuss an Azol verwendet werden.

Die Reaktionstemperaturen können bei den Verfahren (a) und (b) in einem grösseren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmässigerweise beim Siedepunkt des jeweiligen Lösungsmittels gearbeitet.

Bei der Durchführung der erfindungsgemässen Verfahren (a) bzw. (b) setzt man auf 1 mol der Verbindungen der Formel (II) bzw. (IV) vorzugsweise 1 bis 2 mol Pyridinol der Formel (111) bzw. 1 bis 2 mol Azol und jeweils 1 bis 2 mol Säurebinder ein. Zur Isolierung der Verbindungen der Formel (I) wird das Lösungsmittel abdestilliert und der Rückstand entweder mit Wasser versetzt und kräftig gerührt, wobei das Reaktionsprodukt durchkristallisiert, oder mit einem Gemisch aus einem organischen Solvens und Wasser aufgenommen, die organische Phase abgetrennt, mit Wasser gewaschen, über Natriumsulfat getrocknet und im Vakuum vom Lösungsmittel befreit. Der Rückstand wird gegebenenfalls durch Destillation oder Umkristallisation gereinigt.

Für die erfindungsgemässe Umsetzung gemäss Verfahrensvariante (c) kommen als Verdünnungsmittel vorzugsweise polare organische Lösungsmittel infrage. Hierzu gehören vorzugsweise chlorierte Kohlenwasserstoffe, wie Dichloräthan; Alkohole, wie Äthanol, Propanol, n-Butanol; Ketone, wie Aceton, Methyläthylketon, Methylbutylketon; Äther, wie Tetrahydrofuran, Dioxan und Nitrile, wie Acetonitril.

Die Umsetzung nach Verfahren (c) wird in Gegenwart eines Säurebinders vorgenommen. Hierzu gehören vorzugsweise die anorganischen und organischen Säurebindemittel, die bei den Umsetzungen nach den Verfahren (a) und (b) bereits vorzugsweise genannt wurden.

Die Reaktionstemperaturen können beim Verfahren (c) in einem grösseren Bereich variiert werden. Im allgemeinen arbeitet man zwischen 0 und 150°C, vorzugsweise zwischen 50 und 90°C.

Bei der Durchführung des erfindungsgemässen Verfahrens (c) setzt man auf 1 mol Dihalogenketon der Formel (V) vorzugsweise 1 mol Pyridinol, 1 bis 1,2 mol Azol sowie 2 bis 3 mol Säurebindemittel ein. Zur Isolierung der Verbindungen der Formel (I) wird das Lösungsmittel im Vakuum weitgehend abdestilliert. Der Rückstand wird in Gegenwart eines inerten, mit Wasser nicht mischbaren Lösungsmittels, wie beispielsweise Toluol, Xylol oder Dichloräthan, mit wenig verdünnter Salzsäure versetzt, um überschüssiges Azol als Hydrochlorid zu entfernen. Danach wird die organische Phase mit verdünnter Alkalilauge neutral gewaschen und das Lösungsmittel im Vakuum abdestilliert. Der Rückstand wird gegebenenfalls durch Destillation oder Umkristallisation gereinigt. Die erfindungsgemässe Reduktion erfolgt in üblicher Weise, wie z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemässe Umsetzung polare organische Lösungsmittel infrage. Hierzu gehören vorzugsweise Alkohole, wie Methanol, Äthanol, Butanol, Isopropanol, und Äther, wie Diäthyläther oder Tetrahydrofuran. Die Reaktion wird im allgemeinen bei 0 bis 30°C, vorzugsweise bei 0 bis 20°C durchgeführt. Hierzu setzt man auf 1 mol des Ketons der Formel (I) etwa 1 mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemässe Umsetzung bevorzugt Alkohole, wie Isopropanol, oder inerte Kohlenwasserstoffe, wie Benzol, infrage. Die Reaktionstemperaturen können wiederum in einem grösseren Bereich variiert werden; im allgemeinen arbeitet man zwischen 20 und 120°C, vorzugsweise bei 50 bis 100°C. Zur Durchführung der Reaktion setzt man auf 1 mol des Ketons der Formel (I) etwa 1 bis 2 Aluminiumisopropylat ein. Zur Isolierung der reduzierten Verbindungen der Formel (I) wird das überschüssige Lösungsmittel durch Destillation im Vakuum entfernt und die entstandene Aluminiumverbindung mit verdünnter Schwerfelsäure oder Natronlauge zersetzt. Die weitere Aufarbeitung erfolgt in üblicher Weise.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der Formel (I) kommen vorzugsweise Salzevon Metallen der 11. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich physiologisch verträglichen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Äthanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Als Beispiele für besonders wirksame Vertreter der erfindungsgemässen Wirkstoffe seien ausser den Herstellungsbeispielen und den Beispielen der Tabelle I genannt (wobei der Ausdruck azol-1-yl für 1,2,4-triazol-1-yl und imidazol-1-yl steht):
1-(4-Chlorpyridin-2-yloxy)-3,3-dimethyl-1-azol-1-ylbutan-2-on und -ol
1- (5-Chlorpyridin-2-yloxy)-3,3-dimethyl-1-azol-1-ylbutan-2-on und -ol
1-(6-Brompyridin-2-yloxy)-3,3-dimethyl-1-azol-1-ylbutan-2-on und -ol
1-(6-Fluorpyridin-2-yloxy)-3,3-dimethyl-1-azol-1-ylbutan-2-on und -ol
1-(6-Jodpyridin-2-yloxy)-3-3-dimethyl-1-azo)-1-ylbutan-2-on und -ol
1-(4-Brompyridin-2-yloxy)-3,3-dimethyl-1-azol-1-ylbutan-2-on und -ol
1 - -(5-Brompyridin-2-yloxy)3,3-dimethyl-1-azol-1-ylbutan-2-on und -ol
1-(4,6-Dichlorpyridin-2-yloxy)3-3-dimethyl-1-azol-1-ylbutan-2-on und -ol
1-(3,6-Dichlorpyridin-2-yloxy)-3,3-dimethyl-1-azol-1-ylbutan-2-on und -ol
1-(3,5,6-Trichlorpyridin-2-yloxy)-3,3-dimethyl-1-azol-1-ylbutan-2-on und -ol
1-(3,6-Dibrompyridin-2-yloxy)-3,3-dimethyl-1-azol-1-ylbutan-2-on und -ol
1-(3,5,6-Tribrompyridin-2-yloxy)-3,3-dimethyl-1-azol-1-ylbutan-2-on und -ol
1-(4,6-Dimethylpyridin-2-yl-oxy)-3,3-dimethyl-1-azol-1-ylbutan-2-on und -ol
1-(3-Cyanopyridin-2-yloxy)-3,3-dimethyl-1-azol-1-ylbutan-2-on und -ol
1-(6-Cyanopyridin-2-yloxy)-3,3-dimethyl-1-azol-1-ylbutan-2-on und -ol
1-(2-Fluorpyridin-3-yloxy)-3,3-dimethyl-1-azol-1-ylbutan-2-on und -ol
1-(4-Chlorpyridin-3-yloxy)-3,3-dimethyl-1-azol-1-ylbutan-2-on und -ol
1-(5-Chlorpyridin-3-yloxy)-3,3-dimethyl-1-azol-1-ylbutan-2-on und -ol
1-(6-Chlorpyridin-3-yloxy)-3,3-dimethyl-1-azol-1-ylbutan-2-on und -ol
1-(2,6-Dichlorpyridin-2-yloxy)-3,3-dimethyl-1-azol-1-ylbutan-2-on und -ol
1-(2,4,6-Trichlorpyridin-2-yloxy)-3,3-dimethyl-1-azol-1-ylbutan-2-on und -ol
1-(4,5,6-Trichlorpyridin-2-yloxy)-3,3-dimethyl-1-azol-1-ylbutan-2-on und -ol
1-(2,4,5-Trichlorpyridin-2-yloxy)-3,3-dimethyl-1-azol-1-ylbutan-2-on und -ol
1-(4-Bromopyridin-3-yloxy)-3,3-dimethyl-1-azol-1-yl-butan-2-on und -ol
1 - (5-Brompyridin-3-yloxy)-3,3-dimethyl-1-azol-1-ylbutan-2-on und -ol
1-(6-Brompyridin-3-yloxy)-3,3-dimethyl-1-azol-1-ylbutan-2-on und -ol
1-(5-Jodpyridin-3-yloxy)-3,3-dimethyl-1-azol-1-ylbutan-2-on und -ol
1-(6-Jodpyridin-3-yloxy)-3,3-dimethyl-1-azol-1-ylbutan-2-on und -ol
1-(2,6-Dibrompyridin-3-yloxy)-3,3-dimethyl-1 -azol-1 -ylbutan-2-on und -ol
1-(5-Fluorpyridin-3-yloxy)-3,3-dimethyl-1-azol-1-ylbutan-2-on und -ol
1-(6-Fluorpyridin-3-yloxy)-3,3-dimethyl-1-azol-1-ylbutan-2-on und -ol
1-(6-Methylpyridin-3-yloxy)-3,3-dimethyl-1-azol-1-ylbutan-2-on und -ol
1-(2-Chlorpyridin-4-yloxy)-3,3-dimethyl-1-azol-ylbutan-2-on und -ol
1-(2-Brompyridin-4-yl-oxy)-3,3-dimethyl-1-azol-1-ylbutan-2-on und -ol
1-(2,6-Dichlorpyridin-4-yloxy)-3,3-dimethyl-1-azol-1-ylbutan-2-on und -ol
1-(2,6-Dibrompyridin-4-yloxy)-3,3-dimethyl-1-azol-1-ylbutan-2-on und -ol
1-(2,6-Dimethylpyridin-4-yloxyl-3,3-dimethyl-1-azol-1-ylbutan-2-on und -ol
1-(2,6-Difluorpyridin-4-yloxy)-3,3-dimethyl-1-azol-1-ylbutan-2-on und -ol
1-(2-Fluorpyridin-4-yloxy)-3,3-dimethyl-1-azol-1-ylbutan-2-on und -ol

Die erfindungsgemässen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chrytridiomycetes, Zygomycetes, Ascomycetes, Basidiomycetes, Deuteromycetes.

Die erfindungsgemässen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemässen Wirkstoffe mit besonders gutem Erfolg zur Bekämpfung echter Mehltaupilze, wie zur Bekämpfung von Gurkenmehltau (Erysiphe cichoriacearum), Getreidemehltau sowie gegen andere Getreidekrankheiten, wie Getreiderost und die Streifenkrankheit der Gerste verwendet werden; ausserdem auch zur Bekämpfung von Venturiaarten, wie gegen den Erreger des Apfelschorfs (Fusicladium dentriticum), sowie der Pilze Pyridularia und Pellicularia Besonders hervorzuheben ist, dass die erfindungsgemässen Wirkstoffe nicht nur eine protektive Wirkung entfalten, sondern teilweise auch kurativ wirksam sind, also bei Anwendung nach erfolgter Infektion. Weiterhin ist die teilweise systemische Wirkung der Stoffe hervorzuheben. So gelingt es, Pflanzen gegen Pilzbefall zu schützen, wenn man den Wirkstoff über den Boden und die Wurzel oder über das Saatgut den oberirdischen Teilen der Pflanze zuführt.

Als Pflanzenschutzmittel können die erfindungsgemässen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosol, Suspensionsemulsionskonzentrate, Saatgutpuder, Wirkstoff-imprägnierte Natur - und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebelformulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeugende Mittel: Nichtionogene und anionische Emulgatoren, wie Polyoxyäthylen-Fettsäure-Ester, Polyoxyäthylen-Fettalkohol-Äther, z.B. Alkylarylpolyglykoläther, Alkylsulfonate, Alkylsulfate, Arylsulfonate sowie Eiweisshydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azometallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungiziden, 1 nsektiziden, Akariziden, Nematiziden, Herbiziden, Schutzstoffen gegen Vogelfrass, Wuchsstoffen, Pflanzennährstoffen und Bodenstruktur-Verbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Giessen, Spritzen, Sprühen, Stäuben, Streuen, Trockenbeizen, Feuchtbeizen, Nassbeizen, Schlämmbeizen oder Inkrustieren.

Bei der Verwendungs als Blattfungizide können die Wirkstoffkonzentrationen in den Anwendungsformen in einem grösseren Bereich variiert werden. Sie liegen im allgemeinen zwischen 0,1 und 0,00001 Gewichtsprozent. Vorzugsweise zwischen 0,05 und 0,0001 %.

Bei der Saatgutbehandlung werden im allgemeinen Wirkstoffmengen von 0,001 bis 50 g/kg Saatgut, vorzugsweise 0,01 bis 10 g benötigt.

Zur Bodenbehandlung sind Wirkstoffmengen von 1 bis 100 g/cm³ Boden, wie insbesondere 10 bis 200 g, erforderlich.

Die vielseitigen Verwendungsmöglichkeiten gehen aus den nachfolgenden Beispielen hervor.

### Beispiel A:

Erysiphe-Test (Gurken) / systemisch
Lösungsmittel: 4,7 Gewichtsteile Aceton
Emulgator: 0,3 Gewichtsteile Alkylarylpolyglykol- äter
Wasser: 95,0 Gewichtsteile

Man vermischt die für die gewünschte Wirkstoffkonzentration in der Giessflüssigkeit nötige Wirkstoffmenge mit der angegebenen Menge des. Lösungsmittels und verdünnt das Konzentrat mit der angegebenen Menge Wasser, welches die genannten Zusätze enthält.

In Einheitserde angezogene Gurkenpflanzen werden im 1- bis 2-Blattstadium innerhalb einer Woche dreimal mit 10 cm³ Giessflüssigkeit in der angegebenen Wirkstoffkonzentration, bezogen auf 100 cm³ Erde, gegossen.

Die so behandelten Pflanzen werden nach Behandlung mit Konidien des Pilzes Erysiphe cichoriacearum inokuliert. Anschliessend werden die Pflanzen bei 23 bis 24°C und einer relativen Luftfeuchtigkeit von 70% im Gewächshaus aufgestellt. Nach 12 Tagen wird der Befall der Gurkenpflanzen bestimmt. Die erhaltenen Boniturwerte werden in Prozent Befall umgerechnet. 0% bedeutet keinen Befall, 100% bedeutet, dass die Pflanzen vollständig befallen sind.

In diesem Test zeigen z.B. folgende Verbindungen eine sehr gute Wirkung, die derjenigen der aus dem Stand der Technik bekannten Verbindungen deutlich überlegen ist:
Verbindungen gemäss Herstellungsbeispielen 1 und 6.

### Beispiel B:

Fusicladium-Test (Apfel) / protektiv
Lösungsmittel: 4,7 Gewichtsteile Aceton
Emulgator: 0,3 Gewichtsteile Alkylarylpolyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 h bei 20°C und einer relativen Luftfeuchtigkeit von 70% im Gewächshaus. Anschliessend werden sie mit einer wässrigen Konidiensuspension des Apfelschorferregers (Fusicladium dentriticum) inokuliert und 18 h lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

In diesem Test zeigen z.B. folgende Verbindungen eine sehr gute Wirkung, die derjenigen der aus dem Stand der Technik bekannten Verbindungen deutlich überlegen ist:
Verbindungen gemäss Herstellungsbeispielen 1 und 6.

### Beispiel C:

### Sprossbehandlungstest / Getreidemehltau / protektiv kurativ

### (blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Alkylarylpolyglykoläther auf und gibt 975 Gewichtsteile Wasser hinzu. Das Konzentrat verdünnt man mit Wasser auf die gewünschte Endkonzentration der Spritzbrühe. Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Zur Prüfung auf kurative Wirksamkeit geht man in entsprechender Weise aber umgekehrter Reihenfolge vor. Die Behandlung der einblättrigen Gerstenjungpflanzen mit der Wirkstoffzubereitung erfolgt 48 h nach der Inokulation, wenn die Infektion bereits manifest ist.
Nach 6 Tagen Verweilzeit der Pflanzen bei einer Temperatur von 21-22°C und einer Luftfeuchtigkeit von 80-90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

In diesem Test zeigen z.B. folgende Verbindungen eine sehr gute Wirkung, die derjenigen der aus dem Stand der Technik bekannten Verbindungen deutlich überlegen ist:
Verbindungen gemäss Herstellungsbeispielen 1,3 und 6.

### Beispiel D:

### Gerstenmehltau-Test ( Erysiphe graminisvar. hordei) / systemisch

### (pilzliche Getreidesprosskrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22°C und 80-90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer, je geringer der Mehltaubefall ist.

In diesem Test zeigen z.B. folgende Verbindungen eine sehr gute Wirkung, die derjenigen der aus dem Stand der Technik bekannten Verbindungen deutlich überlegen ist:
Verbindung gemäss Herstellungsbeispiel 6.

### Beispiel E:

### Sprossbehandlungstest / Getreiderost / protektiv (blattzerstörende Mykose)

Zur Herstellung einerzweckmässigen Wirkstoffzubereitung nimmt man 0,25 Gewischtsteile Wirkstoff in 25 Gewichtsteilen Dimethylformamid und 0,06 Gewichtsteilen Alkylarylpolyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 h bei etwa 20°C und einer 100%igen Luftfeuchtigkeit in ein Gewächshaus.

Nach 10 Tagen Verweilzeit der Pflanzen bei einer Temperatur von 20°C und einer Luftfeuchtigkeit von 80-90% wertet man den Besatz der Pflanzen mit Rostpusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Rostbefall ist.

In diesem Test zeigen z.B. folgende Verbindungen eine sehr gute Wirkung, die derjenigen der aus dem Stande der Technik bekannten Verbindungen deutlich überlegen ist:
Verbindung gemäss Herstellungsbeispiel 6.

### Beispiel F:

### Saatgutbeizmittel-Test / Streifenkrankheit der Gerste

### (samenbürtige Mykose)

Zur Herstellung eines zweckmässigen Trockenbeizmittels verstreckt man den Wirkstoff mit einem Gemisch aus gleichen Gewichtsteilen Talkum und Kieselgur zu einer feinpulvrigen Mischung mit der gewünschten Wirkstoffkonzentration.

Zur Beizung schüttelt man Gerstensaatgut, das durch Drechslera graminea (Syn. Helminthosporium gramineum) natürliche verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4°C aus. Dabei wird die Keimung der Gerste und gegebenenfalls auch der Pilzsporen eingeleitet. Anschliessend sät man die vorgekeimte Gerste mit 2 x 50 Korn 2 cm tief in Fruhstorfer Einheitserde und kultiviert sie im Gewächshaus bei Temperaturen um 18°C in Saatkästen, die täglich 16 h dem Licht ausgesetzt werden. Innerhalb von 3 bis 4 Wochen bilden sich die typischen Symptome der Streifenkrankheit aus.

Nach dieser Zeit bestimmt man die Anzahl der kranken Pflanzen in Prozent der insgesamt aufgelaufenen Pflanzen. Der Wirkstoff ist um so wirksamer, je weniger Pflanzen erkrankt sind.

In diesem Test zeigen z.B. folgende Verbindungen eine sehr gute Wirkung, die derjenigen der aus dem Stand der Technik bekannten Verbindungen deutlich überlegen ist:
Verbindungen gemäss Herstellungsbeispielen 1 und 6.

### Beispiel G:

Pyricularia- und Pellicularia-Test
Lösungsmittel: 11,75 Gewichtsteile Aceton
Dispergiermittel: 0,75 Gewichtsteile Alkylarylpolyglykoläther
Wasser: 987,50 Gewichtsteile
Andere Zusätze: Gewichtsteile

Man vermischt die für die gewünschte Wirkstoffkonzentration in der Spritzflüssigkeit nötige Wirkstoffmenge mit der angegebenen Menge des Lösungsmittels und des Dispergiermittels undverdünnt das Konzentrat mit der angegebenen Menge Wasser.

Mit der Spritzflüssigkeit bespritzt man etwa 2-4 Wochen alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70%. Danach wird der eine Teil der Pflanzen mit einer wässerigen Suspension von 100000 bis 200000 Sporen/ml von Pyricularia oryzae inokuliert und in einem Raum bei 24 bis 26°C und 100% relativer Luftfeuchtigkeit aufgestellt. Der andere Teil der Pflanzen wird mit einer auf Malzagar gezogenen Kultur von Pellicularia sasakii infiziert und bei 28 bis 30°C sowie 100% relativer Luftfeuchtigkeit aufgestellt.

5 bis 8 Tage nach der Inokulation wird der Befall bei allen zur Zeit der Inokulation mit Pyricularia oryzaevorhandenen Blättern in Prozent der unbehandelten, aber ebenfalls inokulierten Kontrollpflanzen bestimmt. Bei den mit Pellicularia sasakii infizierten Pflanzen wird der Befall nach der gleichen Zeit an den Blattscheiden ebenfalls im Verhältnis zur unbehandelten, aber infizierten Kontrolle bestimmt. Die Auswertung erfolgt in Wertzahlen von 1 -9.1: bedeutet 100%ige Wirkung, 3: gute Wirkung, 5: mässige Wirkung und 9: keine Wirkung. Dabei zeigen z.B. folgende Verbindungen eine sehr gute Wirkung, die derjenigen der aus dem Stand der Technik bekannten Verbindungen deutlich überlegen ist: Verbindungen gemäss Herstellungsbeispielen 3 und 6.

### Herstellungsbeispiele

### Beispiel 1:

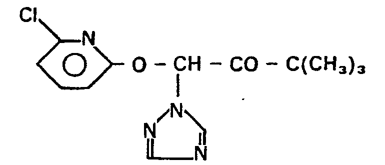

### (Verfahrensvariante a)

123 g (0,5 mol) rohes
1 -Brom-3,3-dimethy)-1-(1,2,4-triazol--yl)-butan-2-on

und 65 g (0,5 mol) 6-Chlor-2-hydroxypyridin werden in 250 ml absolutem Acetonitril unter Rühren bei 24 bis 30°C tropfenweise mit 50,5 g (0,5 mol) Triäthylamin versetzt. Man lässt 3 h bei Raumtemperatur rühren, filtriert und engt das Filtrat im Vakuum ein. Der Rückstand kristallisiert nach dem Behandeln mit Wasser aus. Man erhält 76,6 g (52% der Theorie)
1-(6-Chlorpyridin-2-yloxy)-3,3-dimethyl-1-(1,2,4-triazol-1-yl)-butan-2-on

vom Schmelzpunkt 103-105°C.

### Herstellung des Vorproduktes

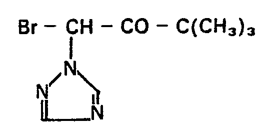

Zu 83,5 (0,5 mol)
3,3-Dimethyl-1-(1,2,4-triazol-1-yl)-butan-2-on und 41 g (0,5 mol)
wasserfreiem Natriumacetat in 250 ml Eisessig werden unter Rühren bei 40 bis 50°C langsam 80 g (0,5 mol) Brom zugetropft. Man lässt bei 40°C bis zur völligen Entfärbung weiterrühren. Danach gibt man die Reaktionsmischung auf 400 ml Wasser und extrahiert dreimal mit je 100 ml Chloroform. Die vereinigten organischen Phasen werden zunächst mit Natriumhydrogencarbonatlösung bis zur Beendigung der CO₂-Entwicklung und anschliessend mit Wasser gewaschen, über Natriumsulfat getrocknet und im Vakuum durch Abdestillieren des Lösungsmittels eingeengt. Man erhält quantitativ rohes
1-Brom-3,3-dimethyl-1-(1,2,4-triazol-1-yl)-butan-2-on,

das direkt weiter umgesetzt wird.

### Herstellung des Ausgangsproduktes:

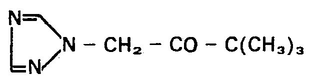

134,5 g (1 mol) 1-Chlor-3,3-dimethylbutan-2-on,
69 g (1 mol) 1,2,4-Triazol

und 140 g (1 mol) gepulvertes Kaliumcarbonat werden in 500 ml Aceton unter Rühren 6 h unter Rückfluss erhitzt. Danach lässt man abkühlen, filtriert das anorganische Salz ab und engt das Filtrat im Vakuum ein. Der ölige Rückstand kristallisiert nach dem Behandeln mit Diisopropyläther aus. Man erhält nach dem Trocknen 123,6 g (74% der Theorie)
3,3-Dimethyl-1-(1,2,4-triazol-1-yl)-butan-2-on vom Schmelzpunkt 63-65°C.

### (Verfahrensvariante b)

Zu einer Lösung von 6,9 g (0,1 mol) 1,2,4-Triazol und 10,1 g (0,1 mol) Triäthylamin in 100 ml Acetonitril gibt man bei 20°C langsam 39,5 g (0,1 mol) rohes
1-Brom-1-(6-chlorpyridin-2-yloxy)-3,3-dimethylbutan-2-on

in 100 ml Acetonitril. Danach lässt man 1 h unter Rückfluss rühren, engt durch Abdestillieren des Lösungsmittels ein und nimmt den Rückstand mit Wasser auf, der dabei kristallisiert. Nach dem Umkristallisieren aus Essigester/Petroläther erhält man 13,5 g (45% der Theorie)
1-(6-Chlorpyridin-2-yloxy)-3,3-dimethyl-1-(1,2,4-triazol-1-yl)-butan-2-on

vom Schmelzpunkt 105°C.

### Herstellung des Vorproduktes:

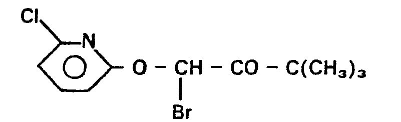

22,7 g (0,1 mol)
1-(6-Chlorpyridin-2-yloxy)-3,3-dimethylbutan-2-on

und 8,2 g (0,1 mol) wasserfreies Natriumacetat werden in 100 ml Eisessig suspendiert und bei 40 bis 50°C langsam mit 16 g (0,1 mol) Brom versetzt. Man lässt bei 40°C bis zur völligen Entfärbung weiterrühren. Danach gibt man die Reaktionsmischung auf 200 ml Wasser und extrahiert zweimal mit je 100 ml Chloroform. Die vereinigten organischen Phasen werden zunächst mit Natriumhydrogencarbonatlösung bis zur Beendigung der CO₂-Entwicklung und anschliessend mit Wasser gewaschen, über Natriumsulfat getrocknet und im Vakuum durch Abdestillieren des Lösungsmittels eingeengt. Man erhält quantitativ rohes
1-Brom-1-(6-chlorpyridin-2-yloxy)-3,3-dimethylbutan-2-on,

das direkt weiter umgesetzt wird.

### Herstellung des Ausgangsproduktes:

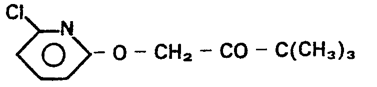

27 g (0,2 mol) Monochlorpinakolin, 26 g (0,2 mol) 6-Chlor-2-hydroxypyridin und 28 g (0,2 mol) gepulvertes Kaliumcarbonat werden in 150 ml Aceton 3 h unter Rühren und unter Rückfluss erhitzt. Danach lässt man abkühlen, filtriert das ausgefallenen Salz ab und engt das Filtrat im Vakuum ein. Der ölige Rückstand wird in Petrol- äther aufgenommen, die Lösung auf -10°C abgekühlt und der dabei ausfallende kristalline Niederschlag abgesaugt und getrocknet. Man erhält 24,5 g (54% der Theorie)
1-(6-Chlorpyridin-2-yloxy)-3,3-dimethylbutan-2-on.

### Verfahrensvariante c)

### 33,8 g (0,2 mol)

1,1-Dichlor-3,3-dimethylbutan-2-on, 26 g (0,2 mol) 6-Chlor-2-hydroxypyridin, 21 g (0,3 mol) 1,2,4-Triazol und 56 g (0,4 mol) Kaliumcarbonat werden in 250 ml Aceton 12 h unter Rühren und unter Rückfluss erhitzt. Danach lässt man abkühlen, filtriert das ausgefallene Salz ab und engt das Filtrat im Vakuum ein. Der ölige Rückstand wird mit Isopropanol versetzt, wobei sich nacheinander drei Kristallfraktionen isolieren lassen. Die ersten beiden enthalten hauptsächlich
1,1-Bis-(1,2,4-triazol-1-yl)-3,3-dimethylbutan-2-on

vom Schmelzpunkt 157-158°C sowie
1,1-Bis-(6-Chlorpyridin-2-yloxy)-3,3-dimethylbutan-2-on

vom Schmelzpunkt 102-104°C. Aus der dritten Fraktion erhält man 4 g
1-(6-Chlorpyridin-2-yloxy)-3,3-dimethyl-1 -(1,2,4-triazol-1-yl)-butan-2-on

vom Schmelzpunkt 102°C

### Beispiel 2:

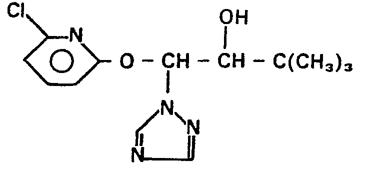

(Reduktion)
11,8 g (0,04 mol) 1-(6-Chlorpyridin-2-yloxy)-3,3-dimethyl-1-(1,2,4-triazol-1-yl)-butan-2-on

(Beispiel 1) werden in 100 ml Methanol gelöst und bei 20-30°C potionsweise unter Rühren mit 1,8 g (0,04 mol) Natriumborhydrid versetzt. Nach Beendigung der exothermen Reaktion werden 5 ml konzentrierte Salzsäure zugetropft und 1 h bei Raumtemperatur gerührt. Danach versetzt man mit 200 ml Wasser und neutralisiert mit Natriumhydrogencarbonatlösung. Das Reaktionsgemisch wird mit Äther ausgeschüttelt, die organische Phase über Natriumsulfat getrocknet und eingeengt. Man erhält 5,3 g (45% der Theorie)
1-(6-Chlorpyridin-2-yloxy)-3,3-dimethyl-1-(1,2,4-triazol-1-yl)-butan-2-ol

als Diastereomerengemisch als zähflüssiges Öl.

### Beispiel 3:

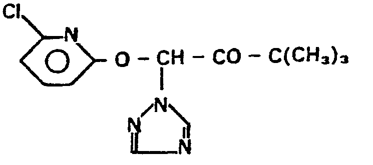

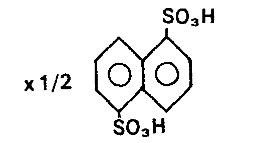

10 g (0,034 mol) 1-(6-Chlorpyridin-2-yloxy)-3,3-dimethy)-1-(1,2,4-triazot-1-yl)-butan-2-on

(Beispiel 1) werden in 50 ml Aceton gelöst und mit einer Lösung von 8 g (0,027 mol) Naphthalin-1,5-disulfonsäure in 50 ml Aceton versetzt. Das nach einiger Zeit ausfallende Salz wird abgesaugt und getrocknet. Man erhält 14g (94% der Theorie) 1-(6-Chlorpyridin-2-yloxy)-3,3-dimethyl-
1-(1,2,4-triazol-1-yl)-butan-2-on-naphthalin-1,5-disulfonat

vom Schmelzpunkt 212-215°C (Zers.).

Analog werden die Beispiele der folgenden Tabelle I erhalten.

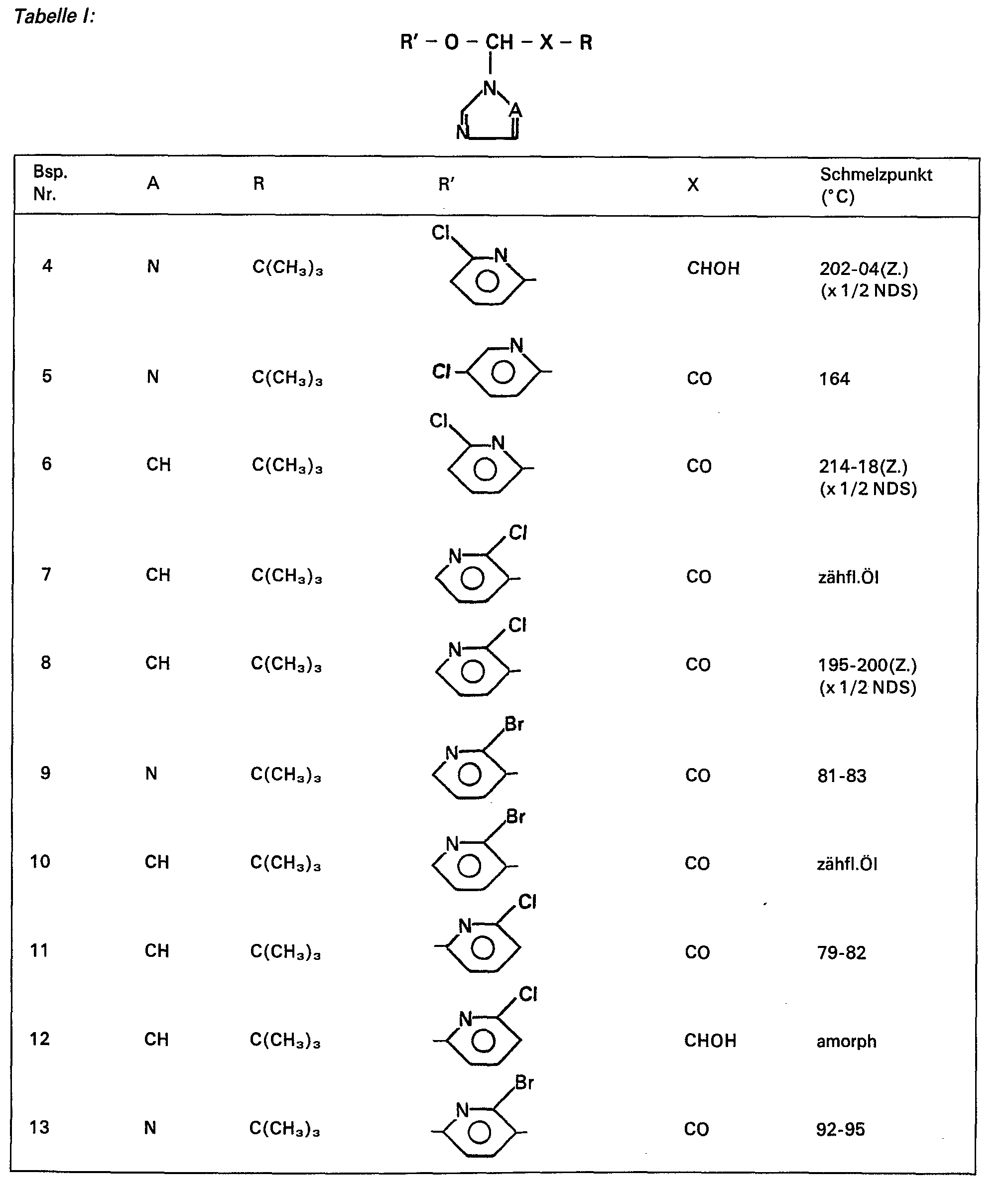

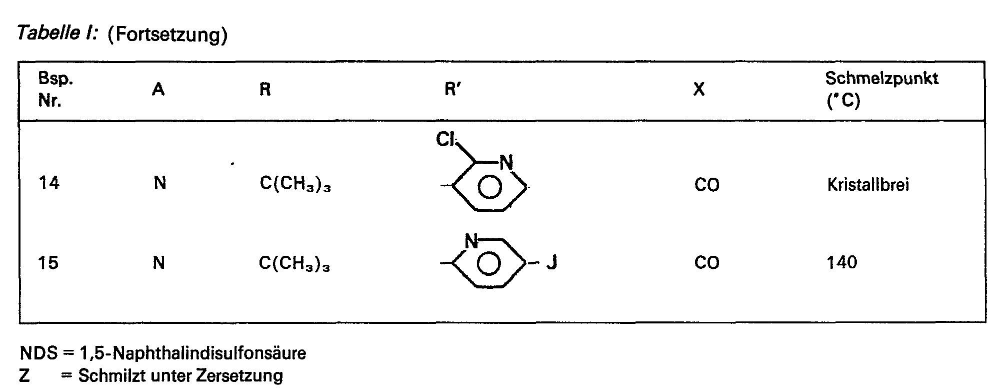

## Patentansprüche

1. Azolylalkylpyridinyläther der Formel: 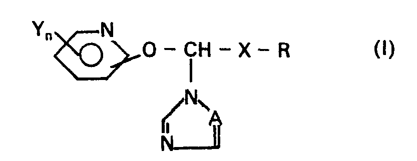 in welcher
Afür die CH-Gruppe oder ein Stickstoffatom steht,
R für Alkyl mit 1 bis 4 Kohlenstoffatomen steht,
X für ein Ketogruppe oder eine CH(OH)-Gruppierung steht,
Y für Halogen, Alkyl mit 1 bis 4 Kohlenstoffatomen oder für Cyano steht, und
n für die Zahlen 0, 1, 2 oder 3 steht,
und deren Säureadditionssalze sowie Metallsalzkomplexe.

2. Verfahren zur Herstellung von Azolylalkylpyridinyläthern, dadurch gekennzeichnet, dass man
a) Azolylhalogenketone der Formel: 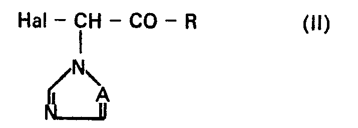 in welcher
A und R die in Anspruch 1 angegebene Bedeutung haben, und
Hal für Chlor oder Brom steht,
mit Pyridinolen der Formel: 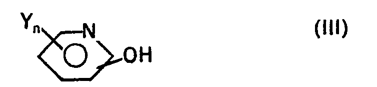 in welcher
Y und n die in Anspruch 1 angegebene Bedeutung haben,
in Gegenwart eines Säurebindemittels und gegebenenfalls
in Gegenwart eines Verdünnungsmittels umsetzt, oder
b) Halogenätherketone der Formel: 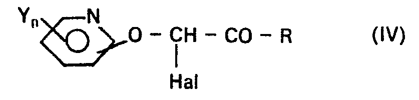 in welcher
R, Y und n die in Anspruch 1 angegebene Bedeutung haben, und
Hal für Chlor oder Brom steht,
mit Imidazol oder 1,2,4-Triazol in Gegenwart eines Säurebindemittels und gegebenenfalls in Gegenwart eines Verdünnungsmittels umsetzt, oder
c) Dihalogenketone der Formel: 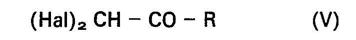 in welcher
R die in Anspruch 1 angegebene Bedeutung hat, und
Hal für Chlor oder Brom steht,
mit Imidazol oder 1,2,4-Triazol und mit einem Pyridinol der Formel (III) in Gegenwart eines Säurebindemittels und gegebenenfalls in Gegenwart eines Verdünnungsmittels umsetzt, und
gegebenenfalls die nach den Verfahrensvariante (a), (b) und (c) erhaltenen Ketoderivate nach bekannten Methoden in üblicher Weise reduziert, und ferner gegegebenenfalls an die so erhaltenen Verbindungen der Formel (I) anschliessend eine Säure oder ein Metallsalz addiert.

3. Fungizide Mittel, gekennzeichnet durch einen Gehalt an mindestens einem Azolylakylpyridinyläther gemäss Anspruch 1.

4. Verwendung von Azolylalkylpyridinyläther gemäss Anspruch 1 zur Bekämpfung von Pilzen.

## Claims

1. Azolylalkylpyridinyl ethers of the formula: 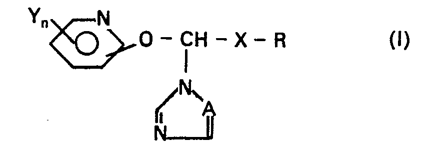 in which
A represents the CH group or a nitrogen atom,
R represents alkyl with 1 to 4 carbon atoms,
X represents a keto group or a CH(OH) grouping,
Y represents halogen, alkyl with 1 to 4 carbon atoms or cyano, and
n represents the numbers 0,1,2 or 3, and their acid addition salts and metal salt complexes.

2. Process for the preparation of azolylalkylpyridinyl ethers, characterised in that:
a) azolylhalogenoketones of the formula: 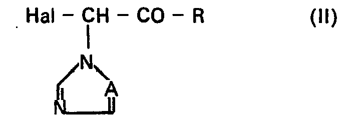 in which
A and R have the meaning indicated in Claim 1, and
Hal represents chlorine or bromine,
are reacted with pyridinols of the formula: 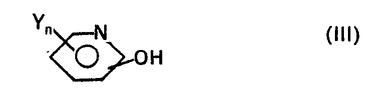 in which
Y and n have the meaning indicated in Claim 1, in the presence of an acid-binding agent and optionally in the presence of a diluent, or
b) halogenoetherketones of the formula: 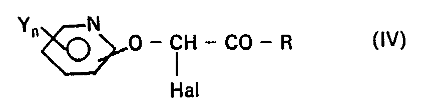 in which
R, Y and n have the meaning indicated in Claim 1, and
Hal represents chlorine or bromine,
are reacted with imidazole or 1,2,4-triazole in the presence of an acid-binding agent and optionally in the presence of a diluent, or
c)dihalogenoketones of the formula: 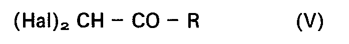 in which
R has the meaning indicated in Claim 1, and
Hal represents chlorine or bromine,
are reacted with imidazole or 1,2,4-triazole and with a pyridinol of the formula (III) in the presence of an acid-binding agent and optionally in the presence of a diluent and the keto derivatives obtained according to process variants a, b and c are optionally reduced by known methods in the customary manner, and furthermore an acid or a metal salt are then optionally added to the compounds of formula (I) thus obtained.

3. Fungicidal agents according to Claim 1, characterised in that they contain at least one azolylalkylpyridinyl ether.

4. Use of azolylalkylpyridinyl ethers according to Claim 1 for combating fungi.

## Revendications

1. Ethers azolylalkylpyridinyliques de formule: 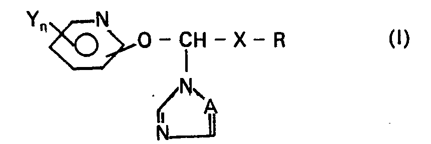 dans laquelle
A représente le groupe CH ou un atome d'azote,
R représente un groupe alkyle contenant 1 à 4 atomes de carbone,
X représente un groupe céto ou un groupement CH(OH),
Y représente un atome d'halogène, un groupe alkyle contenant 1 à 4 atomes de carbone ou un groupe cyano, et
n représente un des nombres 0,1, 2 et 3, ainsi que leurs sels d'addition d'acide et leurs complexes de sels métalliques.

2. Procédé de préparation d'éthers azolylalkylpyridinyliques, caractérisé en ce que:
a) on fait réagir des azolylhalogénocétones de formule: 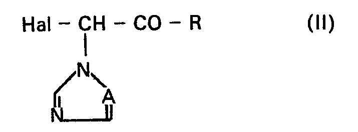 dans laquelle
A et R ont les significations indiquées dans la revendication 1, et
Hal représente un atome de chlore ou un atome de brome,
avec des pyridinols de formule: 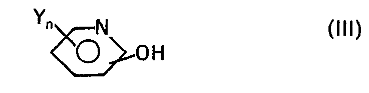 dans laquelle Y et n ont les significations indiquées dans la revendication1,
en présence d'un agent fixateur d'acide et éventuellement en présence d'un diluant, ou
b) on fait réagir des halogénoéthercétones de formule: 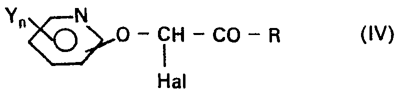 dans laquelle
R, Y et n ont les significations indiquées dans la revendication 1, et
Hal représente un atome de chlore ou un atome de brome,
avec l'imidazole ou le 1,2,4-triazole en présence d'un agent fixateur d'acide et éventuellement en présence d'un diluant, ou
c) on fait réagir des dihalogénocétones de formule: 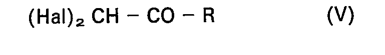 dans laquelle
R a la signification indiquée dans la revendication 1, et
Hal représente un atome de chlore ou un atome de brome,
avec l'imidazole ou le 1,2,4-triazole et avec un pyridinol de formule (III) en présence d'un agent fixateur d'acide et éventuellement en présence d'un diluant, et on réduit éventuellement, de la manière habituelle et selon des procédés connus, les dérivés céto obtenus conformément aux variantes opératoires a, b et c et, en outre, aux composés ainsi obtenus de formule (1), on ajoute ensuite éventuellement un acide ou un sel métallique.

3. Agents fongicides, caractérisés en ce qu'ils contiennent au moins un éther azolylalkylpyridi- nylique suivant la revendication 1.

4. Utilisation d'éthers azolylalkylpyridinyliques suivant la revendication 1 pour combattre les champignons.
